(19) Europäisches Patentamt
European Patent Office
Office européen des brevets

(11) **EP 1 654 195 B1**

(12) **FASCICULE DE BREVET EUROPEEN**

(45) Date de publication et mention de la délivrance du brevet:
**15.06.2016 Bulletin 2016/24**

(21) Numéro de dépôt: **04767622.6**

(22) Date de dépôt: **08.07.2004**

(51) Int Cl.:
***C01B 25/32*** *(2006.01)*    ***A61K 9/20*** *(2006.01)*

(86) Numéro de dépôt international:
**PCT/FR2004/001790**

(87) Numéro de publication internationale:
**WO 2005/007599 (27.01.2005 Gazette 2005/04)**

(54) **PROCÉDÉ DE PREPARATION DE GRANULES DE PHOSPHATES DE CALCIUM DE TYPE HYDROXYAPATITE**

VERFAHREN ZUR HERSTELLUNG VON CALCIUMPHOSPHATKÖRNEN VOM HYDROXYLAPATIT-TYP

METHOD FOR PREPARING HYDROXYAPATITE-TYPE CALCIUM PHOSPHATE GRANULES

(84) Etats contractants désignés:
**AT BE BG CH CY CZ DE DK EE ES FI FR GB GR HU IE IT LI LU MC NL PL PT RO SE SI SK TR**

(30) Priorité: **16.07.2003 FR 0308660**

(43) Date de publication de la demande:
**10.05.2006 Bulletin 2006/19**

(73) Titulaire: **Innophos, Inc.**
**Cranbury, NJ 08512 (US)**

(72) Inventeurs:
• **LEITE, Lorraine**
**F-75020 Paris (FR)**
• **GODBER, John**
**Lawrenceville, 08648 New Jersey (US)**

(74) Mandataire: **Bauer, Wulf et al**
**Patentanwälte**
**Bauer Vorberg Kayser**
**Partnerschaft mbB**
**Goltsteinstrasse 87**
**50968 Köln (DE)**

(56) Documents cités:
**WO-A- 02/089775    US-A- 4 335 086**
**US-A- 5 066 441**

• PONTIER C ET AL: "About the use of stoichiometric hydroxyapatite in compression - incidence of manufacturing process on compressibility" EUROPEAN JOURNAL OF PHARMACEUTICS AND BIOPHARMACEUTICS, ELSEVIER SCIENCE PUBLISHERS B.V., AMSTERDAM, NL, vol. 51, no. 3, mai 2001 (2001-05), pages 249-257, XP004239486 ISSN: 0939-6411
• MONMA H ET AL: "PREPARATION OF HYDROXYAPATITE BY THE HYDROLYSIS OF BRUSHITE" J MATER SCI DEC 1987, vol. 22, no. 12, décembre 1987 (1987-12), pages 4247-4250, XP001161107
• ITOH, HIDEAKI ET AL: "A new porous hydroxyapatite ceramic prepared by cold isostatic pressing and sintering synthesized flaky powder" DENTAL MATERIALS JOURNAL (1994), 13(1), 25-35, 1994, XP008027708
• DATABASE CA [Online] CHEMICAL ABSTRACTS SERVICE, COLUMBUS, OHIO, US; CHENG, MINYUAN ET AL: "Preparation of hydroxyapatite powder by hydrolysis of brushite investigation on active bioceramic" XP002271274 extrait de STN Database accession no. 134:9288 CA & GUANGXI DAXUE XUEBAO, ZIRAN KEXUEBAN (1999), 24(4), 280-283, 1999,

- DATABASE CA [Online] CHEMICAL ABSTRACTS SERVICE, COLUMBUS, OHIO, US; KAWASAKI, TSUTOMU ET AL: "Manufacture of hydroxyapatite crystalline particles for chromatography" XP002271275 extrait de STN Database accession no. 107:201456 CA & JP 62 202808 A (KOKEN CO., LTD., JAPAN;RESEARCH DEVELOPMENT CORP. OF JAPAN) 7 septembre 1987 (1987-09-07)
- DATABASE WPI Section Ch, Week 199511 Derwent Publications Ltd., London, GB; Class A60, AN 1995-077865 XP002271276 & JP 07 002506 A (TAIHEI KAGAKU SANGYO KK) 6 janvier 1995 (1995-01-06)
- "Handbook of Pharmaceutical Fxcipients (Fourth edition)" 2003, SCIENCE AND PRACTICE, ROYAL PHARMACEUTICAL SOCIETY OF GREAT BRITAIN , LONDON, UK , XP002271273 page 78 - page 79

**Description**

L'invention se rapporte à un procédé de préparation d'un excipient de phosphate de calcium et concerne en particulier les phosphates de calcium qui présentent des diagrammes de diffraction aux rayons X caractéristiques du minéral hydroxyapatite.

Plus précisément, l'invention vise en particulier les phosphates de calcium sous forme de granulés présentant un diagramme de diffraction aux rayons X caractéristique de l'hydroxyapatite et de bonnes caractéristiques de compressibilité et d'écoulement dans les applications à compression directe.

L'invention concerne un procédé particulièrement économique de préparation desdits granulés.

PONTIER C ET AL: "About the use of stoichiometric hydroxyapatite in compression-incidence of manufacturing process on compressibility" EUROPEAN JOURNAL OF PHARMACEUTICS AND BIOPHARMACEUTICS, ELSEVIER SCIENCE PUBLISHERS B.V., AMSTERDAM, NL, vol. 51, no. 3, mai 2001 (2001-05), pages 249-257, XP004239486 ISSN: 0939-6411 présente une étude sur l'influence des différents paramètres d'une hydroxyapatite stoechiométrique sur sa compressibilité (calcination, granulation sèche ou humide et taille des granulés), en vue de son utilisation comme excipient dans des comprimés obtenus par compression directe. L'étude a porté sur deux catégories de tailles moyennes de granulés à savoir 200 microns et 400 microns. Cette étude précise par ailleurs que la taille moyenne de 200 microns est la plus communément utilisée dans la compression directe.

ITOH, HIDEAKI ET AL: "A new porous hydroxyapatite ceramic prepared by cold isostatic pressing and sintering synthesized flaky powder" DENTAL MATERIALS JOURNAL (1994), 13(1), 25-35, 1994, XP008027708, divulgue une méthode de préparation d'hydroxyapatite (HAp) à partir de brushite par conversion hydrolytique en deux étapes, à l'aide d'une solution basique (par exemple NH4OH). Cette méthode conduit dans une première étape à une HAp non stoechiométrique puis dans une deuxième étape au produit stoechiométrique. Cette méthode, basée sur la méthode conventionnelle en une étape, a pour propos d'améliorer la productivité.

La mise en forme de matériaux secs sous forme de comprimés est effectuée préférentiellement en faisant appel à la technique de la compression directe.

L'article « Preparation of hydroxyapatite by the hydrolysis of brushite », de H. MONNA et al., Journal of materials science, 22, 4247-4250, 1987, divulgue une méthode de préparation d'hydroxyapatite à partir de brushite par conversion hydrolytique en deux étapes à l'aide d'une solution basique (par exemple $NH_4OH$). Cette méthode conduit dans une première étape à une hydroxyapatite non steochiométrique puis dans une deuxième étape au produit steochiométrique. Cette méthode, basée sur la méthode conventionnelle en une étape, a pour propos d'améliorer la productivité. La mise en forme de matériaux secs sous forme de comprimés est effectuée préférentiellement en faisant appel à la technique de la compression directe.

En effet, la voie de compression directe exige le moins d'étapes de traitement et évite le contact des matières actives avec des solvants potentiellement néfastes comme l'eau ou les solvants organiques.

La réduction du nombre des étapes de traitement et l'absence de mise en oeuvre de solvants diminuent les coûts et permet ainsi de réduire la dégradation des matières actives.

La formation de comprimés par compression directe exige l'utilisation d'un matériau de mise sous forme de comprimés ou d'un véhicule connu sous le terme d'excipient.

L'excipient idéal est celui qui est non réactif vis-à-vis des matières actives dans la formulation.

L'excipient idéal doit également être facilement compressible, en formant des comprimés solides avec des surfaces de comprimés lisses. La résistance du comprimé dans la plupart des applications devrait être aussi élevée que possible pour une force de compression minimale.

L'excipient idéal doit également s'écouler fort bien, sans ségrégation quelconque. Ceci est nécessaire en vue du remplissage des matrices des

machines à comprimer haute vitesse sans risque d'obstruction et sans aucune variation dans le poids du matériau qui remplit les matrices.

La plupart des matériaux pulvérulents sont peu pratiques pour la mise sous forme de comprimés par compression directe dans les machines à comprimer haute vitesse, en raison de problèmes relatifs à l'écoulement.

Ces matériaux pulvérulents peuvent souvent être granulés par des moyens variés, par exemple par agglomération sèche sous pression, compression préalable de la poudre en granulats ou par passage de la poudre entre deux rouleaux de compression.

D'autres méthodes de granulation de poudres sont l'agglomération par voie humide, suivie d'un séchage comme, par exemple, un dispositif d'agglomération à plateau rotatif ou sous forme d'un lit fluidisé.

On peut tout aussi bien utiliser le séchage par atomisation, avec ou sans additif d'agglomération (par exemple PVP).

Le compactage préalable de poudres pour produire des matériaux qui s'écoutent bien a souvent un effet nuisible sur la compressibilité du matériau sous forme de granulats.

Ces étapes supplémentaires dans le traitement de l'excipient ajoutent un surcoût et rendent par la même le procédé moins économique.

Il est connu d'utiliser les phosphates de calcium comme excipients.

Ainsi, sont utilisés comme excipients des phosphates de calcium comme le dihydrate d'hydrogénophosphate de calcium [di-hydrate de phosphate dicaicique, $CaHP0_4-2H_20$; brushite], l'hydrogénophosphate calcique anhydre [phosphate dicalcique anhydre, $CaHP0_4$ : monétite] et l'hydroxyapatite [$Ca_5 (PO_4)_3 (OH)$, auquel l'on fait souvent référence commercialement par le terme de « phosphate tricalcique »].

La formule chimique idéale de l'hydroxyapatite est $Ca_5 (P0_4)_3 (OH)$. Il est toutefois bien connu dans la littérature que le réseau cristallin qui possède cette formule idéale, est extrmement totérant en ce qui concerne les substitutions d'anions et de cations dans le réseau.

La substitution de cations par des éléments comme le magnésium, le strontium, le baryum, le sodium, le plomb et un grand nombre d'autres atomes est bien connue.

La substitution d'anions peut prendre trois formes différentes.

Premièrement, une partie des groupements de phosphate trivalent ($PO_4^{3-}$) peut être remplacée par $HP0_4^{2-}$, ceci conduisant à une apatite non stoechiométrique.

Deuxièmement, les groupements de phosphate trivalent ($PO_4^{3-}$) peuvent être remplacés par d'autres anions complexes comme les carbonates ou les vanadates.

Troisièmement, le groupement hydroxyle ($OH^-$) peut être remplacé partiellement ou complètement par d'autres anions comme le fluorure ou le chlorure.

Une substitution couplée est également bien connue, dans laquelle un ion est remplacé par un autre ion de charge différente et la neutralité de charges est maintenue par des substitutions ailleurs dans les ions du réseau avec des charges différentes ou par des vacances.

Dans l'ensemble de ces substitutions, le facteur qui reste commun et distingue le matériau comme étant une hydroxyapatite est son diagramme caractéristique de diffraction aux rayons X.

Dans le cadre de la présente invention, le terme hydroxyapatite, fait référence substantiellement aux phosphates de calcium qui présentent le diagramme de diffraction aux rayons X de l'hydroxyapatite.

Sans pour autant lier la portée du brevet à une formule, les phosphates peuvent être représenter par la formule suivante $Ca_{5-x} (P0_4)_{3-x} (HP0_4)_x (OH)_{1-x}$ (I) dans ladite formule, x varie entre 0 et 1, de préférence entre 0,1 et 0,5.

Inclut est le cas où de faibles quantités par exemple moins de 5 % en poids, de préférence entre 0,01 et 3 % en poids de calcium sont substitués par un autre cation, en particulier le cation de la base (sodium, potassium).

Inclut est aussi le cas où de faibles quantités de groupements phosphate trivalent ($PO_4^{3-}$) sont substitués par des anions complexes (exemple, carbonate et vanadate) et d'ions hydroxy remplacés par un autre anion, par exemple un halogénure, notamment chlorure ou fluorure.

Les phosphates de calcium conviennent tout à fait bien en tant qu'excipients dans un grand nombre d'applications d'excipients en raison de leurs propriétés intrinsèques.

Ils sont de couleur blanche. Ils sont stables lors du stockage et ne se modifient pas du point de vue chimique au court du temps. Ils sont en général stables vis-à-vis des matières actives et des autres ingrédients présents dans un comprimé.

Les phosphates de calcium sont en général inertes par rapport aux matières actives en raison de leur solubilité très faible dans tous les solvants, tout spécialement dans l'eau.

Ils présentent des profils de désintégration excellents avec les agents de désintégration classiques (croscarmellose sodique) et ils sont compatibles avec une large gamme de matières actives.

Il existe un grand nombre de méthodes de synthèse qui fournissent l'hydroxyapatite en tant que produit final.

Celles-ci sont bien connues dans la littérature.

D'une manière générale, il existe deux voies basse température en vue de la préparation de phosphates de calcium d'hydroxyapatite à savoir la précipitation directe et l'hydrolyse.

Dans la voie de précipitation directe, des sources de calcium et de phosphates sélectionnées d'une manière appropriée entrent en réaction ensemble dans de l'eau pour fournir un phosphate de calcium d'hydroxyapatite.

Le choix des sources de calcium est grand, par exemple l'hydroxyde de calcium, le chlorure de calcium, l'acétate de calcium, le nitrate de calcium sont parmi quelques-uns des matériaux bien connus utilisés dans la précipitation directe de phosphate de calcium d'hydroxyapatite. L'hydroxyde de calcium est préféré.

Les sources de phosphate comprennent l'acide phosphorique, les phosphates de sodium, les phosphates d'ammonium, les phosphates de potassium, parmi d'autres. L'acide phosphorique est préféré.

En général pour la voie de précipitation directe, les matériaux de départ doivent posséder tout simplement une certaine solubilité dans l'eau.

On obtient le phosphate de calcium d'hydroxyapatite par précipitation dans l'eau de la source de calcium et de la source de phosphate.

La voie par hydrolyse se base sur le fait que le phosphate de calcium d'hydroxyapatite est la phase la plus stable du point de vue thermodynamique pour les phosphates de calcium en présence d'eau.

L'hydrolyse du phosphate de calcium du type brushite en solutions aqueuses fournit le phosphate de calcium d'hydroxyapatite.

Un exemple de cette voie est décrit selon US 4 335 086, dans lequel la préparation du phosphate de calcium d'hydroxyapatite est décrite en vue de l'utilisation en chromatographie sur colonne.

Il est également possible d'utiliser la brushite et ta monétité comme excipients.

La brushite et la monétité de phosphates dicalciques peuvent être synthétisées de manière directe sous forme de particules relativement grosses qui présentent de bonnes propriétés d'écoulement.

Ces matériaux présentent également de bonnes caractéristiques de compressibilité dans les applications à compression directe.

Les phosphates de calcium sont produits de la manière la plus économique par la réaction de solutions d'acide phosphorique avec des solutions ou des suspensions d'hydroxyde de calcium.

Par conséquent, par une variation des quantités relatives de calcium et de phosphate selon la stoechiométrie réactionnelle, on peut préparer la monétité ou la brushite.

En contrôlant les vitesses d'addition et les concentrations des réactifs, on peut préparer de grosses particules de monétité et de brushite.

Ces grosses particules présentent un bon écoulement et fournissent une compressibilité élevée.

Le brevet US 5 486 365 enseigne un procédé de granulation du phosphate de calcium de brushite ($CaHP0_4$-$2H_2O$) par séchage par atomisation d'une suspension aqueuse de brushite.

Le phosphate de calcium de brushite est cristallisé dans des conditions spéciales exigeant l'utilisation d'acides organiques pour contrôler la morphologie des cristaux de brushite.

Alors que la brushite et la monétité de phosphates dicalciques sont utiles dans un grand nombre d'applications, elles présentent quelques inconvénients.

La brushite ($CaHP0_4$-$2H_2O$) contient de l'eau faisant partie de sa structure cristalline, qui peut être libérée avec des effets négatifs, soit pendant la préparation des comprimés, soit si le matériau est stocké dans des conditions d'humidité élevée et de chaleur (températures supérieures à 40 °C).

Cette libération potentielle d'eau peut avoir un effet négatif sur les matières actives dans la comprimé.

La monétité ($CaHP0_4$) ne contient pas d'eau, mais possède effectivement une acidité faible qui peut aussi être néfaste à certaines matières actives.

Ces problèmes sont exacerbés dans les processus de granulation par voie humide en raison de la nécessité de l'élimination du solvant, ce qui a pour effet d'exagérer, soit la libération d'eau pour les excipients de brushite, soit l'acidité des excipients de monétite.

Pour ces raisons, on a envisagé d'utiliser un excipient de phosphate de calcium à base d'hydroxyapatite pour éviter tout à la fois le problème de l'eau et des réactions acides avec les matières actives.

Toutefois, par opposition aux phosphates de calcium de brushite et de monétite, il n'est pas possible de produire directement à partir d'hydroxyde de calcium et d'acide phosphorique, de grosses particules de phosphates de calcium d'hydroxyapatite présentant de bonnes propriétés d'écoulement.

L'hydroxyapatite, telle qu'elle est synthétisée à partir d'acide phosphorique et d'hydroxyde de calcium, est bien connue dans l'état de la technique pour être un matériau qui ne fournit pas de bons excipients.

Ceci est lié à deux problèmes dont le premier est la difficulté d'obtenir directement et économiquement de grosses particules d'hydroxyapatite.

Ceci est pris en compte par l'addition d'étapes de traitement supplémentaires coûteuses comme la granulation à rouleaux ou le séchage par atomisation.

Le deuxième problème se rapporte au fait qu'une fois de grosses particules obtenues, le produit, tout en étant compressible, exige des forces de compactage très élevées pour fournir des comprimés non friables.

Alors que les origines de ce fait ne sont pas comprises, et ne désirant pas être associé à une théorie quelconque, l'on pense que ces problèmes sont liés à la méthode selon laquelle l'hydroxyapatite est produite.

Par conséquent, l'hydroxyapatite est coûteuse à produire sous forme de granulés qui possède un bon écoulement et le matériau qui est produit ne présente pas de bonnes caractéristiques de compressibilité.

Il a été proposé afin de surmonter les problèmes de la compressibilité de l'hydroxyapatite, de préparer des mélanges intimes d'hydroxyapatite (auxquels l'on fait référence par le terme de phosphate tricalcique dans l'état de la technique) avec d'autres matériaux.

C'est ainsi que le brevet US 3 987 204 enseigne que l'hydroxyapatite, à elle seule, n'est pas un excipient utile, mais qu'en ajoutant des quantités substantielles de farine de caroube au phosphate de calcium d'hydroxyapatite, l'on peut produire un matériau directement compressible.

Ce processus met en jeu l'utilisation d'autres constituants et un traitement coûteux et prolongé de mélange et alors de séchage du produit hydroxyapatite/farine de caroube.

L'addition de cellulose microcristalline à l'hydroxyapatite est décrite dans le brevet US 4 781 925 pour améliorer les caractéristiques de compressibilité. L'objet de ce brevet est d'incorporer des agents de désintégration dans les comprimés d'hydroxyapatite en vue d'une utilisation en tant que supplément de calcium.

La cellulose microcristalline est bien connue pourfournir des caractéristiques de compressibilité excellentes, mais cette dernière est chère et son utilisation exige une étape de mélange supplémentaire dans le processus.

Par conséquent, il est souhaitable de posséder un phosphate de calcium d'hydroxyapatite pour des raisons de compatibilité de l'excipient avec les matières actives, tout en ayant, en même temps, une hydroxyapatite qui présente de bonnes caractéristiques d'écoutement et de compressibilité et qui soit accessible à raison d'un coût modeste.

Ce type de matériau n'a jamais été mis à disposition dans l'état de la technique.

Un objectif de la présente invention est de fournir un excipient qui soit utile dans une large gamme d'applications, et qui puisse être utilisé dans des processus de fabrication des comprimés par compression directe ou par granulation par voie humide.

Un autre objectif de la présente invention est de fournir un excipient utile dans les procédés à compression directe qui possède une compressibilité améliorée.

Un objectif supplémentaire de l'invention est de fournir un excipient de phosphate de calcium amélioré approprié à la compression directe.

Un objectif supplémentaire est de fournir un excipient de phosphate de calcium à écoulement libre qui a des caractéristiques supérieures de compressibilité et qui soit approprié aux applications à compression directe.

Un autre objectif de la présente invention est de fournir un excipient qui soit économique en termes de coûts de production.

Les granulés obtenus selon l'invention présentent des caractéristiques physico-chimiques précisées ci-après.

Les définitions et les méthodes de détermination des caractéristiques données ci-après, sont précisées dans les exemples.

Les granulés sont de couleur blanche.

Ainsi, la taille de particules peut s'échelonner entre 1 et 500 $\mu$m.

Plus précisément, au moins 90 % en poids des particules sont supérieures à 10 microns et 90 % en poids des particules inférieures à 300 microns, de préférence inférieures à 260 microns.

Précisons que la détermination des tailles se fait par passage sur des tamis métalliques. Généralement, la taille des particules exprimée par le diamètre médian ($d_{50}$) est comprise entre 100 $\mu$m et 250 $\mu$m, de préférence entre 150 $\mu$m et 190 $\mu$m. On définit le diamètre médian comme étant tel que 50 % en poids des particutes ont un diamètre supérieur ou inférieur au diamètre médian.

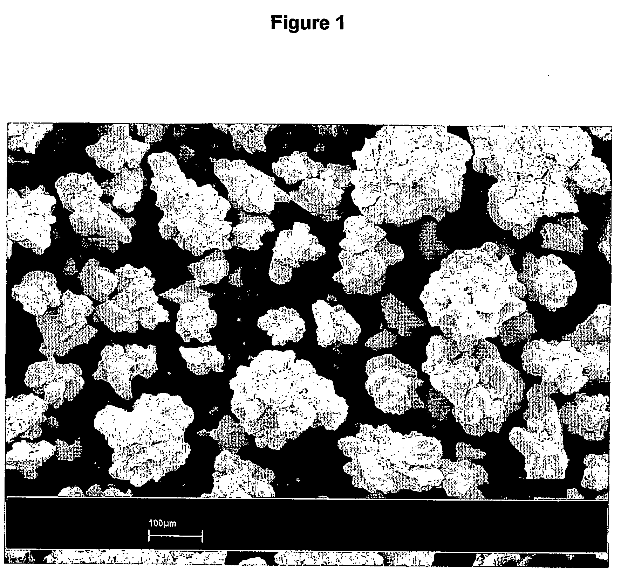

La figure 1 représente une photographie prise au microscope électronique à balayage qui illustre la morphologie de type granulés du phosphate de calcium d'hydroxyapatite obtenu selon l'invention.

Les granulés de phosphate de calcium d'hydroxyapatite ont une densité qui peut être plus ou moins élevée. La densité apparente (non tassée) des granulés est de préférence, d'au moins 0,6 et se situe encore plus préférentiellement entre 0,6 et 1,0, de préférence entre 0,68 et 0,72.

La densité apparente (tassée) des granulés est de préférence, d'au moins 0,7 et se situe encore plus préférentiellement entre 0, 7 et 1,1, de préférence entre 0,76 et 0, 82.

Ils présentent avantageusement une surface spécifique BET comprise entre 10 et 100 m$^2$/g, de préférence comprise entre 50 et 80 m$^2$/g.

Les granulés de phosphate de calcium obtenus selon l'invention présentent une cohésion adaptée à de bonnes propriétés d'écoulement.

L'indice d'écoulement mesuré en instantané est toujours largement supérieur à 10.

Les granulés de phosphate de calcium d'hydroxyapatite ont des caractéristiques de compressibilité supérieures par rapport à d'autres phosphates de calcium.

Ainsi, le profil de compressibilité peut être défini ainsi :

- de 15 à 40 KPa pour une compresssion de 30 KN,
- de 10 à 25 KPa pour une compresssion de 20 KN,
- de 3 à 10 KPa pour une compresssion de 10 KN.

On obtient des granulés de phosphate de calcium ayant une forme physique leur permettant de résister à l'attrition, qui conservent une porosité interne importante et, de ce fait, une vitesse de dissolution rapide, lors de leur utilisation.

On précisera, que la vitesse de désintégration dans l'eau, des granulés est inférieure à 60 s, de préférence, inférieure à 25 s et encore plus

préférentiellement comprise entre 5 et 20 s. Les valeurs données correspondent à celles obtenues en mettant en oeuvre le test USP 26 (2040), « Disintegration and dissolution of nutrional suppléments » de la Pharmacopée américaine.

La structure originale des produits est obtenue grâce à un procédé de fabrication parfaitement adapté.

L' objet de l'invention est le procédé de préparation dudit phosphate de calcium d'hydroxyapatite sous forme de granulés ayant de bonnes caractéristiques d'écoulement et de compressibilité, caractérisé par le fait qu'il comprend le traitement d'une suspension de phosphate dicalcique de brushite ayant une taille de particules telles que 90 % en poids d'entre elles soient inférieures à 300 microns, de préférence inférieures à 260 microns et que 90 % en poids d'entre elles soient supérieures à 10 microns, à l'aide d'une solution basique, le maintien du pH a au moins 7,0, pendant une période de temps suffisante pour permettre la transformation du phosphate de calcium de brushite en phosphate de calcium d'hydroxyapatite.

Préférablement, l'hydrolyse est effectuée par chauffage d'une suspension aqueuse à une température et pendant une période de temps suffisante pour permettre le maintien du pH à un niveau stable.

Selon un premier mode de réalisation qui est préféré, on chauffe la suspension aqueuse à la température réactionnelle choisie puis l'on introduit la base tout en régulant le pH.

Selon une autre variante, on ajoute d'abord la base de façon à réguler le pH puis l'on chauffe le milieu à la température réactionnelle choisie.

L'invention peut être mieux comprise par référence à l'équation générale suivante pour l'hydrolyse alcaline de la brushite en hydroxyapatite :

$$5 \ CaHPO_4\text{-}2H_2O + 4 \ MOH + H_2O \rightarrow Ca_5 \ (PO_4)_3 \ (OH) + 2 \ M_2HPO_4 + 14 \ H_2O \qquad \text{Equation [I]}$$

dans laquelle M est le cation apporté par la base, de préférence un cation alcalin, par exemple $Na^+, K^+, NH^{4+}$. Le pH est maintenu à une vateur d'au moins 7,0, de préférence compris entre 7 et 10 et plus préférentiellement compris entre 8 et 8, 5.

La présente invention est une amélioration par rapport à l'état de la technique en ce que le phosphate dé calcium d'hydroxyapatite présentant de bonnes caractéristiques d'écoulement et de compressibilité est obtenu directement par le procédé décrit ci-dessus.

La nouvelle forme de présentation sous forme de granulés du phosphate de calcium d'hydroxyapatite conduit à de nombreux avantages lorsque lesdits granulés sont utilisés comme excipients. Ainsi, ils présentent une capacité de compactage plus élevée par compression directe ce qui, à son tour, fournit des comprimés plus durs, moins friables et réduit l'utilisation de liants et par là-même les coûts, réduit la taille des comprimés, réduit l'énergie requise pour obtenir une dureté de comprimé désirée. De plus, ils permettent la mise en oeuvre des matières actives médiocrement compatibles avec la brushite et la monétite. En raison d'un écoulement amélioré des granulés, ceux-ci conduisent à une meilleure uniformité de composition des comprimés obtenus, permettant des vitesses de compression plus élevées et permettant la mise en oeuvre des médicaments ou des matières actives à écoulement médiocre.

Alors qu'il est bien connu (selon US 4 335 086) que l'hydroxyapatite peut être préparée par l'intermédiaire de l'hydrolyse des hydrogénophosphates de calcium, on a constaté de manière inattendue que grâce à une sélection de la granulométrie du phosphate de calcium de brushite de départ, l'hydroxyapatite ainsi formée par hydrolyse alcaline possède des caractéristiques de compression supérieures tout à la fois au phosphate de calcium de brushite de départ et à d'autres matériaux d'hydroxyapatite produits à partir de procédés différents.

Ce nouveau phosphate de calcium d'hydroxyapatite peut être préparé en partant d'un phosphate de calcium de brushite préparé par un procédé connu quelconque qui prépare le phosphate de calcium de brushite comme défini ici.

Pour obtenir un phosphate de calcium d'hydroxyapatite qui a de bonnes caractéristiques d'écoulement, celui-ci doit avoir selon l'invention, une répartition granulométrique telle que 90 % en poids des particules soient inférieures à environ 300 microns, de préférence inférieures à 260 microns et qu'au moins 90 % en poids des particules soient supérieures à environ 10 microns.

Pour ce faire, le matériau de départ de phosphate de calcium de brushite a une répartition granulométrique telle que 90 % en poids des particules soient inférieures à environ 300 microns, de préférence inférieures à 260 microns et qu'au moins 90 % en poids des particules soient supérieures à environ 10 microns.

Cette répartition granulométrique peut être obtenue par élimination des particules à l'extérieur de ce domaine.

L'opération de sélection granulométrique est effectuée par tamisage.

Dans un mode de réalisation préféré, la taille des particules exprimée par le diamètre médian ($d_{50}$) est compris entre 100 $\mu$m et 250 $\mu$m, de préférence entre 150 $\mu$m et 190 $\mu$m.

De plus, puisque le produit final hydroxyapatite doit être conforme aux réglementations gouvernant l'utilisation de constituants pharmaceutiques, le phosphate de calcium de brushite devrait également respecter les exigences de pureté relatives aux constituants pharmaceutiques, telles qu'elles sont détaillées dans la pharmacopée.

Ainsi, les spécifications pharmaceutiques européennes du phosphate de calcium de brushite dans le cas d'un emploi dans le domaine pharmaceutique sont telles que la teneur en $CaHPO_4, 2H_2O$ est comprise entre 98,0 et 105,5 % en poids et que la teneur en ions chlorure est inférieure ou égale à 330 ppm ; la teneur en ions fluorure inférieure ou égale à 100 ppm ; la teneur en arsenic inférieure ou égale à 10 ppm ; des teneurs en métaux lourds et fer respectivement inférieures ou égales à 40 ppm et 400 ppm.

Conformément au procédé de l'invention, intervient une base pour effectuer la réaction d'hydrolyse qui peut être choisie parmi celles qui sont facilement solubles dans l'eau et qui sont compatibles en vue d'une utilisation avec des constituants pharmaceutiques dans le sens où ils n'introduisent pas de matériau non désiré quelconque qui pourrait contaminer le produit final et qui pourrait le rendre inapproprié à une utilisation par rapport aux exigences de la Pharmacopée.

L'utilisation des bases NaOH, NH$_4$OH et KOH est appropriée à la réaction d'hydrolyse.

On fait appel avantageusement à une solution aqueuse basique ayant une concentration comprise entre 20 et 40 % en poids.

Dans la réaction du phosphate de calcium de brushite avec une solution aqueuse alcaline, il est préférable de garder le pH dans le domaine de 7,0 à 10,0 et plus préférablement dans le domaine de 8,0 à 8,5 pendant la réaction d'hydrolyse.

Il est possible d'effectuer l'hydrolyse à des valeurs de pH inférieures à 8,0, mais la réaction progresse plus rapidement au sein du domaine précité.

Le maintien du pH à 7 nécessite une durée d'hydrolyse plus longue mais cette dernière se poursuit néanmoins.

Sans l'addition d'une base, l'unique produit qui est produit à partir du phosphate de calcium de brushite est le phosphate de calcium de monétite.

Il est avantageux d'effectuer la réaction à une température supérieure à la température ambiante (le plus souvent comprise entre 15°C et 25°C), de préférence supérieure à environ 50°C et encore plus préférentiellement comprise entre 60°C et 100°C. La température idéale se situe aux environs de 90°C.

Aux températures inférieures à 50°C, on a constaté que la réaction nécessite une durée plus longue.

La réaction d'hydrolyse peut être effectuée à l'aide de toute concentration de suspension aqueuse de brushite.

La brushite est maintenue en suspension pendant l'hydrolyse pour garantir l'obtention de granulés homogènes.

Les réactifs sont mis en réaction de préférence avec une agitation suffisante afin de maintenir la brushite en suspension aqueuse.

En pratique, il est difficile de maintenir la brushite en suspension lorsque la concentration est supérieure à environ 50 % en poids. Elle est avantageusement comprise entre 30 et 40 % en poids.

Une agitation exagérée n'améliore pas la vitesse de réaction et peut conduire à une fractionnement des particules avec une perte correspondante en rendement utile.

La quantité de base mise en oeuvre est voisine de celle définie par la stoechiométrie de l'équation [1]. Ainsi, la base peut être utilisée dans une quantité telle qu'elle représente de 80 à 110 % de la quantité stoechiométrique exprimée par rapport au phosphate de calcium de brushite.

Il est possible d'ajouter la totalité de la base au début de la réaction, mais dans le procédé préféré de l'invention, la solution basique est ajoutée optimalement d'une manière progressive, c'est-à-dire au fur et à mesure de la progression de la réaction tout en maintenant la valeur de pH dans la zone prédéfinie.

La solution basique peut être ajoutée par toute méthode quelconque commode, soit directement dans la suspension aqueuse, soit en dérivant une fraction du milieu en formant une boucle de circulation et en introduisant la base au niveau de la boucle de dérivation.

La mesure du pH est effectuée par toute méthode convenable, soit en plaçant un détecteur de pH directement dans la suspension aqueuse, soit en mettant le détecteur de pH dans la ligne de dérivation précédemment mentionnée.

Une fois que le pH est resté stable pendant une période de temps appropriée, approximativement 30 minutes, la transformation du phosphate de calcium de brushite en phosphate de calcium d'hydroxyapatite est complète et le solide peut être séparé de la solution aqueuse.

Ceci peut être accompli de préférence par filtration ou centrifugation. Le solide est lavé à l'aide d'eau et est alors séché.

Le lavage est conduit à l'eau mise en oeuvre en une proportion telle qu'elle représente généralement deux fois le volume du gâteau.

Le séchage est effectué le plus souvent à l'air, de préférence par chauffage du phosphate de calcium d'hydroxyapatite à une température comprise entre 80 et 120 °C, de préférence aux environs de 110 °C pour éliminer l'humidité absorbée par voie physique.

Le phosphate de calcium d'hydroxyapatite qui est préparé par le procédé de l'invention est très pur, comme on le constate en cas de mesure par diffraction aux rayons X.

Les granulés de phosphate d'hydroxyapatite obtenus selon la présente invention peuvent être utilisés dans le domaine pharmaceutique.

Les applications des granulés obtenus selon l'invention sont celles du phosphate de calcium.

De plus, ils présentent l'avantage d'apporter un supplément de calcium et de phosphore dans l'alimentation.

Lesdits éléments jouent un rôle important dans la constitution et le fonctionnement des nerfs, des os, des muscles, des dents.

Les granulés obtenus selon l'invention présentent notamment l'avantage d'tre directement utilisables pour formuler des matières actives par compression directe.

Par «matière active», on entend tout produit destiné à l'administration par voie orale ayant un effet bénéfique ou désiré sur l'utilisateur.

Ainsi, la « matière active » peut être tout produit ayant des propriétés pharmacologiques c'est-à-dire ayant une action préventive ou curative sur un organisme vivant.

On inclut également les produits relevant de la para-pharmacie comme par exemple les vitamines ou les apports d'oligo-éléments minéraux susceptibles d'tre mis sous la forme de comprimés.

Comme exemples de matières actives de type thérapeutique, on peut citer à titre non limitatif les anti-rhumatismaux et anti-inflammatoires non stéroïdiens (kétoprofène, ibuprofène, flurbiprofène, indométacine, phénylbutazone, allopurinol, nabumétone...), les analgésiques opiacés ou non (paracétamol, phénacétine, aspirine...), les antitussifs (codéine, codéthyline, alimémazine...), les psychotropes (trimipramine, amineptine, chlorpromazine et dérivés des phénothiazines, diazépam, lorazépam, nitrazépam, méprobamate, zopiclone, et dérivés de la famille des cyclopyrrolones...), les stéroïdes (hydrocortisone, cortisone, progestérone, testostérone, prednisolone, triamcinolone, dexaméthazone, betaméthazone, paraméthazone, fluocinolone, béclométhazone ...), les barbituriques (barbital, allobarbital, phénobarbital, pentobarbital, amobarbital...), les agents antimicrobiens (péfloxacine, sparfloxacine, et dérivés de la classe des quinolones, tétracylines, synergistines, métionidazole...), les médicaments destinés au traitement des allergies, notamment les antiasthmatiques, les antispasmodiques et antisécrétoires (oméprazole), les vasodilatateurs cérébraux (quinacaïnol), oxprénolol, propranolol, nicergoline.....), les protecteurs cérébraux, les protecteurs hépatiques, les agents thérapeutiques à visée gastro-intestinale, les agents contraceptifs, les vaccins oraux, les agents antihypertenseurs et les agents cardiovasculaires ou cardioprotecteurs tels que les bèta-bloquants et les dérivés nitrés.

La quantité de matière (s) active (s) entrant dans les comprimés préparés selon le procédé de la présente invention peut varier dans de larges limites. Elle est plus particulièrement comprise entre 0,001 et 95 % en poids de la composition totale, le complément étant assuré par la matrice.

Interviennent donc comme constituants principaux de la matrice, les granulés de phosphate de calcium d'hydroxyapatite obtenus selon l'invention.

Le phosphate de calcium d'hydroxyapatite forme en général entre 10 % et 100 % en poids de la matrice. Elle représente avantageusement au moins 80 % et de préférence au moins 90 % en poids de la matrice.

On ajoute avantageusement aux granulés, un agent lubrifiant tel que le stéarate de magnésium, selon une quantité qui est en général de l'ordre de 0,5 % en poids.

On peut également ajouter aux granulés, un agent désintégrant pour favoriser le déliment ultérieur des comprimés. Il peut s'agir d'amidon, notamment de l'amidon de maïs ou du croscarmellose sodique, incorporé selon une quantité pouvant varier entre 5 et 10 % en poids.

La matrice peut comporter en outre un ou plusieurs excipients pharmaceutiquement acceptables, plus particulièrement des agents diluants, des agents de cohésion, des agents lubrifiants et des agents colorants et des arômes tels que les saccharides, notamment le lactose et le saccharose, les acides gras tels que l'acide stéarique, par exemple ; le polyéthylèneglycol ; d'autres phosphates tels que le phosphate dicalcique, la silice, les silicoaluminates, les dérivés cellulosiques notamment l'HMPC, la gomme Xanthane, la gélatiné, la polyvinylpyrrolidone.

Les granulés obtenus selon l'invention sont mélangés avec la ou les matières actives et éventuellement les autres excipients de la composition, selon toute méthode de mélange solide/solide connue et comprimés à sec par compression directe, c'est-à-dire sans utilisation d'eau ou d'un solvant organique tel que l'éthanol.

L'opération de compression consécutive au mélange des excipients et du ou des matières actives est généralement mise en oeuvre sous une force pouvant aller de 6 à 30 kN (mesure au niveau du galet de compression) et de préférence de l'ordre de 10 à 20 kN. Cette opération de compression est, de préférence, précédée d'une pré-compression sous une force pouvant aller de 0,5 à 2,5 kN.

De grandes vitesses de compression peuvent être atteintes grâce au procédé selon l'invention, sans pour autant altérer la qualité des comprimés. Il est notamment possible d'atteindre des vitesses supérieures à 150 000 comprimés/heure, sans entraîner de clivage.

Les comprimés obtenus selon l'invention présentent l'avantage de pouvoir libérer rapidement le principe actif mais aussi de bonnes propriétés mécaniques, notamment de friabilité.

Les comprimés obtenus présentent une friabilité mesurée selon la méthode référencée par la Pharmacopée Américaine USP 26 sous le n 1216 inférieure à 1 %.

Le temps de désintégration mesuré selon la méthode référencée par la Pharmacopée Américaine USP 26 sous le n 2040 est inférieur à 1 minute.

De manière à illustrer plus complètement la nature du procédé et la façon de le mettre en pratique, on donne des exemples de réalisation de l'invention 1 à 4.

L'exemple 5 est un exemple comparatif où le phosphate de calcium d'hydroxyapatite est préparé par précipitation directe suivie d'un compactage préalable sous forme de granulats.

Avant de détailler les exemples, on précise les méthodes utilisées pour la détermination des différentes caractéristiques des produits obtenus.

- la densité apparente tassée et non tassée :

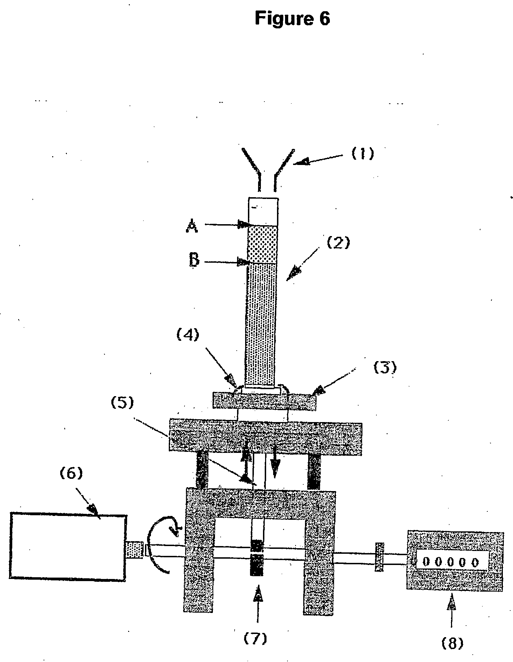

On la mesure sur un appareil illustré par la figure 6.

On commence par peser l'éprouvette vide (2).

On introduit dans l'éprouvette (2) la poudre à mesurer à l'aide de l'entonnoir (1), de manière à ce que le haut du lit de poudre vienne au ras du haut de l'éprouvette jaugée à 250 cm$^3$(niveau A).

On détermine la masse de la poudre par pesée de l'éprouvette pleine.

On assujettit l'éprouvette sur le support (3) par l'intermédiaire de pinces (4).

On met à zéro le compteur (8) qui totalise le nombre de coups imposés au fond de l'éprouvette.

L'éprouvette est soumise à des chocs verticaux appliqués à sa base par l'intermédiaire d'un marteau (5) actionné par un moteur (6) via une came (7). On arrête l'opération lorsque le volume obtenu est constant (niveau B).

On enregistre l'évolution du volume apparent lu sur les graduations de l'éprouvette en fonction du nombre de coups appliqués à l'aide d'un marteau.

On obtient une courbe expérimentale de tassement.

Volume apparent = f (nombre de coups) que l'on transforme en une courbe densité apparente = f (nombre de coups).

On détermine la densité apparente selon la retation :

$$\text{densité apparente} = \frac{\text{masse de la poudre introduite (g)}}{\text{volume apparent (cm}^3)}$$

- la granulométrie :

Elle est mesurée par diffraction de la lumière laser dans les suspensions aqueuses sans ultrasons et sans agent dispersant, par utilisation d'un analyseur granulométrique Beckman Coulter LS230™, en utilisant la théorie de Mie.

- la surface spécifique BET :

La superficie dans tous les exemples est mesurée selon la norme NF ISO 9277, par adsorption d'azote en utilisant la méthode BET à un point et un dispositif d'analyse de surface Micromeritics Flowsorb™. Avant la mesure, tous les échantillons sont soumis à un séchage à l'air dans une étuve, 2 heures à la température de 200°C.

- la capacité d'écoulement :

La capacité d'écoulement dans tous les exemples est mesurée selon un test effectué à l'aide d'un appareillage Van-Kel™ Flowmeter model (VK10210).

Le principe du test consiste à faire écouler 200 grammes de matériau à travers une filière à comprimés du type "B" "7/16". On définit le temps qu'il faut pour écouler les 200 g de matériau. La coulabilité est exprimée en termes de débit soit en g/s.

- la dureté des comprimés :

Les comprimés sont testés, pour ce qui est de la dureté, par utilisation d'un duromètre pour comprimés Schleuniger Tablet Hardness Tester™, modèle 28/106.

Le principe consiste à mettre le comprimé entre les mâchoires de compression et d'exercer une force croissante jusqu'à ce que le comprimé soit écrasé.

La force est enregistrée et exprime la dureté du comprimé.

La dureté indiquée est la moyenne de la dureté de 10 comprimés.

EXEMPLES

Exemple 1

On charge à température ambiante dans un réacteur à double enveloppe dans laquelle circule de l'eau, un phosphate dicalcique de brushite ayant une taille de particules telle que 90 pour cent d'entre elles sont inférieures à 260 microns et que 90 pour cent d'entre elles sont supérieures à 10 microns (produit DITAB, fabriqué par la Société Rhodia) en une quantité telle que l'on obtienne une suspension aqueuse ayant une concentration de 40 % en poids.

On agite la suspension au moyen d'un agitateur mécanique et l'on chauffe à 60°C par l'intermédiaire de la circulation d'eau chaude.

Le pH de la réaction est régulé à 8,0 à l'aidé d'un régulateur de pH.

Dès que le pH est inférieur à cette valeur, le pH est remonté par addition d'une solution aqueuse d'hydroxyde d'ammonium à 20 % exprimé en poids de $NH_3$.

On maintient le chauffage à une température de 60°C.

Après 4 heures, la suspension est refroidie et le solide résultant est séparé de la solution par filtration.

Le solide est lavé à l'aide d'eau et est alors séché à une température de 110°C.

Le produit obtenu a une répartition granulométrique de 90 pour cent inférieure à 260 microns et de 90 pour cent supérieure à 10 microns.

D'autres caractéristiques du produit sont rassemblées dans le tableau (I) donné plus loin.

Exemple 2

On effectue le même traitement, tel décrit dans l'exemple 1, à l'exception du maintien de la température à 90°C.

Exemple 3

On effectue le même traitement, tel décrit dans l'exemple 2, à l'exception de ce que la base utilisée est une solution aqueuse à 20 % en poids d'hydroxyde de sodium.

Exemple 4

On effectue le même traitement, tel décrit dans l'exemple 3, à l'exception de ce que la base utilisée est une solution aqueuse à 30 % en poids d'hydroxyde de potassium.

Exemple comparatif 5

On prépare un phosphate de calcium d'hydroxyapatite selon un procédé classique qui consiste à charger dans un réacteur, une suspension d'hydroxyde de calcium à 12 % (poids/poids), à charger la suspension à une température de 60°C et alors à ajouter une solution de $H_3PO_4$ à 20 % en poids à la suspension de chaux, jusqu'à ce que le pH de la suspension résultante soit compris entre 6 et 7.

On filtre la suspension sur filtre à vide et l'on sèche à une température de 110°C dans un four pendant 8 heures.

On prépare des granulats en alimentant le produit sec ainsi obtenu un système Fitzpatrick Chilsonator™, équipé de rouleaux de 10 cm de large et de 75 cm de diamètre.

Les rouleaux ont une surface revêtue de cannelures sinusoïdales et sont séparés par un interstice de rouleaux de 0,05 cm.

On alimente le mélange pulvérulent par l'intermdiaire d'une bande transporteuse au dispositif de compactage du Chilsonator et qui est soumis à compactage par passage entre les rouleaux.

Un rouleau est forcé par voie hydraulique contre l'autre avec une pression de 70 kg/centimètre carré (pression manométrique).

La force de roulement (roll force) est d'environ 2143 kg par centimètre linéaire. Les rouleaux ont une vitesse de rotation de 16 tours par minute.

Le produit sort sous forme d'une feuille qui est fractionnée à l'aide d'un dispositif de broyage Ftizmill™, (modèle DAS06), équipé de lames de couteaux rotatives.

Le produit est déchargé du dispositif de broyage à travers un tamis qui a des ouvertures rondes de 0,125 cm.

Le produit compacté et broyé est alors directement alimenté à une unité de tamisage par vibrations.

Les tamis utilisés ont un diamètre de 120 cm.

Le premier tamis est classé à 36 TBC,"tensile bolting cloth" (soit une ouverture de maille de 541 $\mu$m) et le deuxième tamis en dessous est classé à 78 TBC (soit une ouverture de maille de 231 $\mu$m).

On sépare ainsi à l'aide de ces tamis de vibration la charge d'alimentation en trois portions.

On récupère la fraction médiane des particules, c'est-à-dire l'ensemble des particules qui passent à travers le tamis 36 TBC, mais qui ne peuvent pas passer à travers le tamis 78 TBC.

Les fractions supérieures et inférieures qui émergent des tamis de vibration sont évacuées en direction de la trémie d'alimentation du Chilsonator, mélangées à la charge d'alimentation brute pour le Chilsonator et sont ainsi recyclées.

Caradéristiques des granulés.

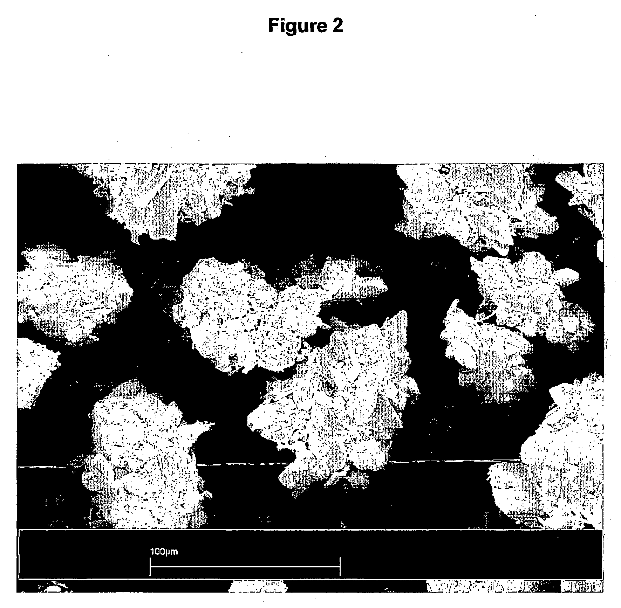

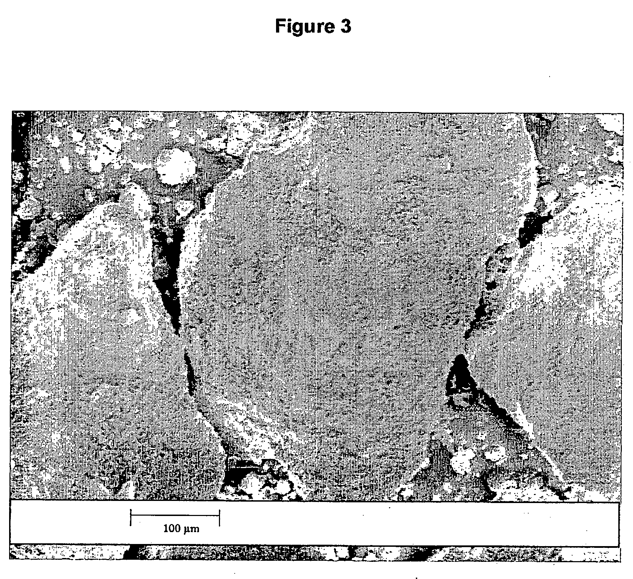

Les figures 1 à 3 données en annexe représentent des photographies prises au microscope électronique à balayage qui illustrent la morphologie des granulés phosphate de calcium d'hydroxyapatite selon l'exemple 2 (figure 1), celle de la matière première à savoir le phosphate de calcium de brushite (figure 2) et la morphologie d'un phosphate de calcium d'hydroxyapatite préparé selon la technique antérieure décrite dans l'exemple 5 (figure 3).

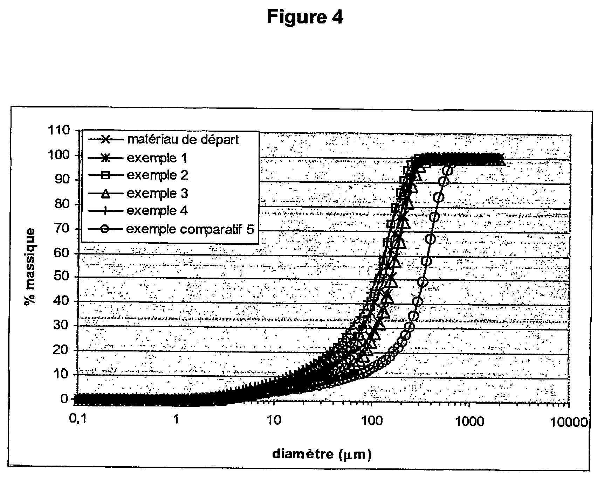

La figure 4 représente un graphique qui correspond aux courbes cumulées pour la détermination du diamètre médian ($d_{50}$) des différents exemples.

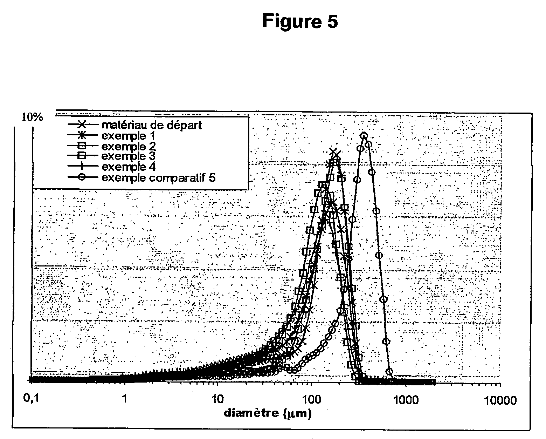

La figure 5 représente un graphique qui illustre la répartition granulométrique des différents exemples.

Les autres caractéristiques physico-chimiques des produits provenant des exemples sont décrites dans le tableau (I).

Tableau (I)

| Matériau | Densité apparente $g/cm^3$ | Capacité d'écoulement g/s | Surface spécifique $m^2/g$ |
|---|---|---|---|
| Matériau de départ | 0,870 | 33 | 2 |
| Exemple 1 | 0,675 | 10,4 | 60 |
| Exemple 2 | 0,721 | 11,9 | 53 |
| Exemple 3 | 0,720 | 11,1 | 20 |
| Exemple 4 | 0,932 | 13,3 | 78 |
| Exemple comparatif 5 | 0,872 | 16,7 | 70 |

Caractéristiques des comprimés.

Des comprimés issus des exemples ci-dessus sont préparés en plaçant le phosphate de calcium d'hydroxyapatite (à raison de 97 pour cent), 2 % en poids d'agent de désintégration Ac-Di-Sol™ (croscarmellose stéarate) et 0,5 % en poids d'agent lubrifiant de stéarate de magnésium dans un malaxeur en V à double coque (Patterson Kelley™), équipé d'une barre d'intensification.

Le mélange est soumis à la procédure de mélange pendant 2 minutes avec la barre d'intensification à l'arrêt.

Les formulations sont mises sous forme de comprimés par compression directe sur une machine à comprimer rotative (Manesty™ B3B), équipée d'un outillage à coupe standard IPT de 7/16 de pouce.

La machine à comprimer est équipée de tensiomètres attachés à un enregistreur afin d'enregistrer la force de compression appliquée lors de chaque lot de comprimés.

On utilise 4 des 16 matrices de la machine à comprimer.

Les comprimés sont produits à une vitesse de 750 comprimés par minute sur la base de 16 matrices.

Le poids nominal des comprimés est de 675 mg.

Les caractéristiques de dureté des comprimés obtenus après compression sur la machine précitée du matériau de départ à savoir le phosphate de calcium de brushite, des granulés de l'invention obtenus selon les exemples 1 à 4 ainsi que du phosphate de calcium d'hydroxyapatite comparatif de l'exemple 5, sont rassemblées dans le tableau (II) qui suit.

Tableau (II)

| Force de compression (kN) | Dureté du matériau de départ (kPa) | Dureté de l'exemple 1 (kPa) | Dureté de l'exemple 2 (kPa) | Dureté de l'exemple 3 (kPa) | Dureté de l'exemple 4 (kPa) | Dureté de l'exemple comparatif 5 (kPa) |
|---|---|---|---|---|---|---|
| 10 | 3 | 6,3 | 3,8 | 8,5 | 4,1 | 1,8 |
| 20 | 7,7 | 17,8 | 16,5 | 21,7 | 14,4 | 4,2 |
| 30 | 15,2 | 31,5 | 19,8 | 39,6 | 23,6 | - |

De ce tout qui précède, l'on peut constater que le produit préparé conformément à la présente invention (exemples 1-4) présente des propriétés de compressibilité qui sont supérieures d'une manière significative à celles du matériau préparé par une voie classique (exemple 5) tout comme des propriétés de compressibilité supérieures à celles du matériau de départ [Tableau (II)].

Les propriétés d'écoulement des produits préparés conformément à la présente invention sont également bonnes, comme il ressort du tableau (I).

De plus, la totalité des comprimés préparés conformément à la présente invention (exemples 1-4) présentent de bons profils de désintégration, avec une vitesse de dissolution de moins d'une minute.

## Revendications

1.  Procédé de préparation d'un phosphate de calcium sous forme de granulés manifestant un diagramme de diffraction aux rayons X d'hydroxyapatite, et comprenant au moins 90 % en poids des particules supérieures à 10 microns et 90 % en poids des particules inférieures à 300 microns, le procédé comprend le traitement d'une suspension de phosphate dicalcique de brushite ayant une taille de particules telles que 90 % en poids d'entre elles soient inférieures à 300 microns, et que 90 % en poids d'entre elles soient supérieures à 10 microns, à l'aide d'une solution basique, le maintien du pH à au moins 7,0, pendant une période de temps suffisante pour permettre la transformation du phosphate de calcium de brushite en phosphate de calcium d'hydroxyapatite.

2.  Procédé selon la revendication 1, **caractérisé par le fait que** la taille des particules du phosphate dicalcique de brushite est telle que le diamètre médian (d5o) est compris entre 100um et 250 $\mu$m, de préférence entre 150 $\mu$m et 190$\mu$m.

3.  Procédé selon la revendication 1, **caractérisé par le fait que** la base utilisée est choisie parmi : NaOH, KOH, NH40H.

4.  Procédé selon la revendication 1, **caractérisé par le fait que** le pH de la réaction d'hydrolyse du phosphate de dicalcique de brushite avec une solution aqueuse alcaline, est maintenu constant entre 7,0 et 10,0 et de préférence entre 8, 0 et 8,5.

5.  Procédé selon la revendication 1, **caractérisé par** le fait la température de la réaction est supérieure à la température ambiante, de préférence supérieure à environ 50 °C et encore plus préférentiellement comprise entre 60 °C et 100 °C.

6.  Procédé selon la revendication 5, **caractérisé par** le fait la température de la réaction se situe aux environs de 90 °C.

7.  Procédé selon la revendication 1, **caractérisé par le fait que** la quantité de base mise en oeuvre est telle qu'elle représente de 80 à 110 % de la quantité stoechiométrique exprimée par rapport au phosphate de calcium de brushite.

8.  Procédé selon la revendication 1, **caractérisé par le fait que** l'on chauffe la suspension aqueuse à la température réactionnelle choisie puis l'on introduit la base tout en régulant le pH.

9.  Procédé selon la revendication 1, **caractérisé par le fait que** l'on ajoute d'abord la base de façon à réguler le pH puis l'on chauffe le milieu à la température réactionnelle choisie.

10. Procédé selon l'une des revendications 8 et 9, **caractérisé par le fait que** la solution basique est ajoutée d'une manière progressive, c'est-à-dire au fur et à mesure de la progression de la réaction tout en maintenant la valeur de pH dans la zone prédéfinie.

**11.** Procédé selon la revendication 1, **caractérisé par le fait que** le phosphate de calcium d'hydroxyapatite est séparé de la solution aqueuse, de préférence par filtration ou centrifugation.

**12.** Procédé selon la revendication 1, **caractérisé par le fait que** le phosphate de calcium d'hydroxyapatite est séché à une température comprise entre 80 et 120 °C, de préférence aux environs de 110 °C.

**Patentansprüche**

**1.** Verfahren zur Herstellung eines Calciumphosphats in Form von Granulat, das ein Hydroxylapatit-Röntgenbeugungsmuster erkennen lässt, und das wenigstens 90 Gew.-% Teilchen größer als 10 Mikrometer und 90 Gew.-% Teilchen kleiner als 300 Mikrometer umfasst, wobei das Verfahren die Behandlung einer Suspension von Brushit-Dicalciumphosphat mit einer solchen Teilchengröße, dass 90 Gew.-% von ihnen kleiner als 300 Mikrometer sind und dass 90 Gew.-% von ihnen größer als 10 Mikrometer sind, mit Hilfe einer basischen Lösung, das Halten des pH-Wertes auf wenigstens 7,0, über einen Zeitraum, der ausreichend ist, um die Umwandlung des Brushit-Calciumphosphats in Hydroxylapatit-Calciumphosphat zu ermöglichen, umfasst.

**2.** Verfahren nach Anspruch 1, **dadurch gekennzeichnet, dass** die Größe der Teilchen des Brushit-Dicalciumphosphats derart ist, dass der mittlere Durchmesser (d50) im Bereich zwischen 100 $\mu$m und 250 $\mu$m, vorzugsweise zwischen 150 $\mu$m und 190 $\mu$m liegt.

**3.** Verfahren nach Anspruch 1, **dadurch gekennzeichnet, dass** die verwendete Base aus NaOH, KOH, NH4OH ausgewählt ist.

**4.** Verfahren nach Anspruch 1, **dadurch gekennzeichnet, dass** der pH-Wert der Hydrolysereaktion des Brushit-Dicalciumphosphats mit einer alkalischen wässrigen Lösung zwischen 7,0 und 10,0 und vorzugsweise zwischen 8,0 und 8,5 konstant gehalten wird.

**5.** Verfahren nach Anspruch 1, **dadurch gekennzeichnet, dass** die Temperatur der Reaktion höher als die Umgebungstemperatur ist, vorzugsweise höher als etwa 50 °C ist und weiterhin bevorzugt im Bereich zwischen 60 °C und 100 °C liegt.

**6.** Verfahren nach Anspruch 5, **dadurch gekennzeichnet, dass** die Temperatur der Reaktion bei etwa 90 °C liegt.

**7.** Verfahren nach Anspruch 1, **dadurch gekennzeichnet, dass** die verwendete Menge an Base derart ist, dass sie 80 bis 110 % der bezogen auf das Brushit-Calciumphosphat ausgedrückten stöchiometrischen Menge ausmacht.

**8.** Verfahren nach Anspruch 1, **dadurch gekennzeichnet, dass** die wässrige Suspension auf die gewählte Reaktionstemperatur erhitzt wird, anschließend die Base unter gleichzeitiger Regelung des pH-Wertes eingeleitet wird.

**9.** Verfahren nach Anspruch 1, **dadurch gekennzeichnet, dass** zunächst die Base zugegeben wird, um den pH-Wert zu regeln, anschließend das Medium auf die gewählte Reaktionstemperatur erhitzt wird.

**10.** Verfahren nach einem der Ansprüche 8 und 9, **dadurch gekennzeichnet, dass** die basische Lösung schrittweise zugegeben wird, das heißt in dem Maße wie die Reaktion fortschreitet, und gleichzeitig der pH-Wert in dem vordefinierten Bereich gehalten wird.

**11.** Verfahren nach Anspruch 1, **dadurch gekennzeichnet, dass** das Hydroxylapatit-Calciumphosphat von der wässrigen Lösung abgetrennt wird, vorzugsweise durch Filtration oder Zentrifugieren.

**12.** Verfahren nach Anspruch 1, **dadurch gekennzeichnet, dass** das Hydroxylapatit-Calciumphosphat bei einer Temperatur im Bereich zwischen 80 und 120 °C, vorzugsweise um die 110 °C, getrocknet wird.

**Claims**

1. A method for the preparation of a calcium phosphate in the form of granules that exhibit a hydroxyapatite x-ray diffraction pattern and that comprise at least 90 % by wt. of particles larger than 10 microns and 90% by wt. of particles smaller than 300 microns, wherein the method comprises treating a brushite dicalcium phosphate suspension having a particle size such that 90% by wt. of them are smaller than 300 microns and 90% by wt. of them are larger than 10 microns with a basic solution, maintaining the pH value at least at 7,0 over a period of time sufficient to permit the transformation of the brushite calcium phosphate into hydroxyapatite calcium phosphate.

2. The method according to claim 1, **characterised in that** the size of the particles of the brushite dicalcium phosphate is such that the mean diameter (d50) is in the range between 100 $\mu$m and 250 $\mu$m, preferably between 150 $\mu$m and 190 $\mu$m.

3. The method according to claim 1, **characterised in that** the base used is selected from NaOH, KOH, $NH_4OH$.

4. The method according to claim 1, **characterised in that** the pH value of the hydrolysis reaction of the brushite dicalcium phosphate suspension with an alkaline aqueous solution is kept constant between 7,0 and 10,0, and preferably between 8,0 and 8,5.

5. The method according to claim 1, **characterised in that** the temperature of the reaction is higher than the ambient temperature, preferably higher than about 50°C, and more preferably is in the range between 60°C and 100°C.

6. The method according to claim 5, **characterised in that** the temperature of the reaction is about 90°C.

7. The method according to claim 1, **characterised in that** the quantity of the base used is such that it amounts to 80 to 110% of the stoichiometric quantity expressed with respect to the brushite calcium phosphate.

8. The method according to claim 1, **characterised in that** the aqueous suspension is heated to the chosen reaction temperature and then the base is introduced while simultaneously regulating the pH value.

9. The method according to claim 1, **characterised in that** first the base is added in order to regulate the pH value, and then the medium is heated to the chosen reaction temperature.

10. The method according to any one of the claims 8 and 9, **characterised in that** the basic solution is added gradually, that is, to the same extent as the reaction progresses, and the pH value is simultaneously maintained within the predefined range.

11. The method according to claim 1, **characterised in that** the hydroxyapatite calcium phosphate is separated from the aqueous solution, preferably by means of filtration or centrifugation.

12. The method according to claim 1, **characterised in that** the hydroxyapatite calcium phosphate is dried at a temperature in the range between 80 and 120°C, preferably around 110°C.

**Figure 1**

**Figure 2**

## Figure 3

100 µm

# Figure 4

# Figure 5

# Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4335086 A
- US 5486365 A
- US 3987204 A
- US 4781925 A
- US 262040 A
- US 2040 A

**Littérature non-brevet citée dans la description**

- About the use of stoichiometric hydroxyapatite in compression-incidence of manufacturing process on compressibility. **PONTIER C et al.** EUROPEAN JOURNAL OF PHARMACEUTICS AND BIOPHARMACEUTICS. ELSEVIER SCIENCE PUBLISHERS B.V, Mai 2001, vol. 51, 249-257
- **ITOH, HIDEAKI et al.** A new porous hydroxyapatite ceramic prepared by cold isostatic pressing and sintering synthesized flaky powder. *DENTAL MATERI-ALS JOURNAL (1994),* 1994, vol. 13 (1), 25-35
- **H. MONNA et al.** *Journal of materials science,* 1987, vol. 22, 4247-4250